(19) **Europäisches Patentamt European Patent Office Office européen des brevets**

(11) **EP 2 504 707 B1**

(12) **FASCICULE DE BREVET EUROPEEN**

(45) Date de publication et mention de la délivrance du brevet:
**13.06.2018 Bulletin 2018/24**

(21) Numéro de dépôt: **10796145.0**

(22) Date de dépôt: **23.11.2010**

(51) Int Cl.:
*G01N 33/68* (2006.01)     *G01N 33/74* (2006.01)

(86) Numéro de dépôt international:
**PCT/IB2010/055380**

(87) Numéro de publication internationale:
**WO 2011/061726 (26.05.2011 Gazette 2011/21)**

(54) **UTILISATION D'UNE ISOFORME D'HLA-G COMME MARQUEUR DE L'OSTEOGENESE**

VERWENDUNG EINER ISOFORM VON HLA-G ALS OSTEOGENESEMARKER

USE OF AN ISOFORM OF HLA-G AS AN OSTEOGENESIS MARKER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.11.2009 FR 0905624**

(43) Date de publication de la demande:
**03.10.2012 Bulletin 2012/40**

(73) Titulaires:
  • **Commissariat à l'Énergie Atomique et aux Énergies Alternatives 75015 Paris (FR)**
  • **Etablissement Français du Sang 93218 La Plaine Saint Denis Cedex (FR)**

(72) Inventeurs:
  • **DESCHASEAUX, Frédéric 37000 Tours (FR)**
  • **SENSEBE, Luc 37300 Joue Les Tours (FR)**
  • **ROUAS-FREISS, Nathalie 75010 Paris (FR)**
  • **NAJI, Abderrahim Carlsbad, CA 92008 (US)**
  • **CAROSELLA, Edgardo Delfino 75116 Paris (FR)**

(74) Mandataire: **Gevers & Orès 41 avenue de Friedland 75008 Paris (FR)**

(56) Documents cités:
**WO-A1-2004/090161     WO-A2-2007/104724 WO-A2-2008/110578**

  • **ROMAN ANGELA ET AL: "Heterogeneous expression of HLA-G1,-G2,-G5,-G6, and-G7 in myeloid and plasmacytoid dendritic cells isolated from umbilical cord blood", HUMAN IMMUNOLOGY, vol. 70, no. 2, février 2009 (2009-02), pages 104-109, XP002585319, ISSN: 0198-8859**

EP 2 504 707 B1

SEQUENCE LISTING

<110> COMMISSARIAT A L'ENERGIE ATOMIQUE ETABLISSEMENT FRANÇAIS DU SANG DESCHASEAUX, Frédéric SENSEBE, Luc ROUAS-FREISS, Nathalie NAJI, Abderrahim CAROSELLA , Edgardo Delfino

<120> Utilisation d'une isoforme d'HLA-G comme marqueur de l'ostéogénèse

<130> F026300371/PCT

<150> FR 09 05624
<151> 2009-11-23

<160> 24

<170> PatentIn version 3.5

<210> 1
<211> 22
<212> DNA
<213> Artificial

<220>
<223> Amorce

<400> 1
aatcccatca ccatcttcca gg          22

<210> 2
<211> 22
<212> DNA
<213> Artificial

<220>
<223> Amorce

<400> 2
agaggcaggg atgatgttct gg          22

<210> 3
<211> 24
<212> DNA
<213> Artificial

<220>
<223> Amorce

<400> 3
tttccagttc agggcagtag tgac          24

<210> 4
<211> 24
<212> DNA
<213> Artificial

<220>
<223> Amorce

<400> 4
cttccccttc ttctccattg tctc          24

<210> 5
<211> 20
<212> DNA

<213> Artificial

<220>
<223> Amorce

<400> 5
ctggacctcg ttgacacctg          20

<210> 6
<211> 20
<212> DNA
<213> Artificial

<220>
<223> Amorce

<400> 6
gacattctct cgttcaccgc          20

<210> 7
<211> 22
<212> DNA
<213> Artificial

<220>
<223> Amorce

<400> 7
tcatgatgct gttgtaggtg gt          22

<210> 8
<211> 20
<212> DNA
<213> Artificial

<220>
<223> Amorce

<400> 8
ctgttccagc catccttcat          20

<210> 9
<211> 21
<212> DNA
<213> Artificial

<220>
<223> Amorce

<400> 9
acatctgcgt ccacatcagg g          21

<210> 10
<211> 22
<212> DNA
<213> Artificial

<220>

<223> Amorce

<400> 10
ccgttcacga gtctcattgg tg        22

<210> 11
<211> 21
<212> DNA
<213> Artificial

<220>
<223> Amorce

<400> 11
atccctgcca gaaccaccac t        21

<210> 12
<211> 23
<212> DNA
<213> Artificial

<220>
<223> Amorce

<400> 12
gcgcttctca ttctcatgga tct       23

<210> 13
<211> 21
<212> DNA
<213> Artificial

<220>
<223> Amorce

<400> 13
atggcgtcct ccctgcttga g        21

<210> 14
<211> 24
<212> DNA
<213> Artificial

<220>
<223> Amorce

<400> 14
aggggtgtgt catgtccaga gagg      24

<210> 15
<211> 24
<212> DNA
<213> Artificial

<220>
<223> Amorce

<400> 15

ccttttcaat ctgagctctt cttt 24

<210> 16
<211> 20
<212> DNA
<213> Artificial

<220>
<223> Amorce

<400> 16
ggaagaggag acacggaaca 20

<210> 17
<211> 21
<212> DNA
<213> Artificial Sequence

<220>
<223> Amorce

<400> 17
atcccatcac catcttccag g 21

<210> 18
<211> 21
<212> DNA
<213> Artificial Sequence

<220>
<223> Amorce

<400> 18
gaggcaggga tgatgttctg g 21

<210> 19
<211> 22
<212> DNA
<213> Artificial Sequence

<220>
<223> Amorce

<400> 19
ggcccacaaa tctcagatcg tt 22

<210> 20
<211> 20
<212> DNA
<213> Artificial Sequence

<220>
<223> Amorce

<400> 20
cactggcgct gcaacaagac 20

<210> 21

<211> 20
<212> DNA
<213> Artificial Sequence

<220>
<223> Amorce

<400> 21
gccaccaacc agccagagaa          20

<210> 22
<211> 25
<212> DNA
<213> Artificial Sequence

<220>
<223> Amorce

<400> 22
gcgaggtact gagtcttctg aaacc          25

<210> 23
<211> 21
<212> DNA
<213> Artificial Sequence

<220>
<223> Amorce

<400> 23
acatggacca gcagactggc a          21

<210> 24
<211> 20
<212> DNA
<213> Artificial Sequence

<220>
<223> Amorce

<400> 24
tcactgtctt gccccaggct          20


**Description**

La présente invention est relative à un nouveau marqueur de l'ostéogenèse, et son utilisation dans des méthodes d'évaluation de l'ostéogenèse chez un mammifère.

L'ostéogenèse est le processus par lequel se forme et se développe le tissu osseux. L'os est formé à partir :

- d'une matrice extracellulaire osseuse (environ 22% à 25%), qui est une matrice organique composée essentiellement de collagène de type I, mais aussi d'autres protéines telles que l'ostéonectine et l'ostéocalcine,
- d'une matrice minérale (environ 70%) très riche en calcium,
- de deux types de cellules osseuses : les cellules ostéoblastiques (ostéoblastes, ostéocytes et cellules bordantes, qui dérivent des cellules souches mésenchymateuses) et les ostéoclastes (cellules dérivant des cellules souches hématopoïétiques), et
- d'eau (5% à 8%).

Les ostéoblastes sont des cellules épithélioïdes mononucléées de forme cubique ou cylindrique, présents dans le tissu osseux en croissance. Ils expriment, de façon caractéristique, le collagène de type I, la phosphatase alcaline (PA ou ALPL), le récepteur 1 de l'hormone parathyroïdienne (PTHR1), l'ostéonectine (SPARC), l'ostéocalcine, le facteur de transcription osterix (OSX) et l'a-actine (ASMA) (Cohen, 2006).

Les ostéocytes sont des ostéoblastes différenciés, très ramifiés et capables de se diviser. Ils assurent le maintien de la matrice extracellulaire osseuse.

Les cellules bordantes sont des cellules au repos, de forme aplatie et allongée, qui se situent à la surface de l'os, dans des zones non-actives c'est-à-dire ni en formation osseuse, ni en résorption osseuse. Elles peuvent, si elles sont stimulées, se différencier en ostéoblastes.

Les ostéoclastes sont des cellules multi-nucléées, de 20 à 100 $\mu$m de diamètre. Ils sont responsables de la résorption osseuse.

Disposer de marqueurs de l'ostéogenèse est important à la fois pour évaluer les risques de fracture chez les personnes atteintes d'une dégénérescence du squelette (par exemple ostéoporose), pour le suivi de la reconstruction post-fracture du squelette, et pour le suivi du développement des tumeurs osseuses. Cette évaluation des risques et ces suivis, s'ils sont précoces, sont indispensables pour mettre en place une thérapie efficace.

A titre d'exemple, 10 à 30 % des adultes ayant une fracture du tibia présentent une pseudarthrose, c'est-à-dire une absence de consolidation des deux fragments osseux survenant après la fracture. En France, cela représente environ 3000 cas par an. Les traitements des pseudarthroses sont extrêmement lourds (autogreffe, injection de moelle osseuse, injection de facteurs de croissance, etc.) et coûteux, et ne permettent pas aux patients de retrouver une autonomie rapidement. Le coût annuel d'un défaut de consolidation du tibia peut être estimé entre 3 et 30 millions d'euros en France. Une détection précoce de la pseudarthrose permettrait donc de traiter plus efficacement les patients atteints de cette affection.

Il existe actuellement sur le marché peu de marqueurs de la formation osseuse (ostéogenèse). Ces marqueurs sont essentiellement la phosphatase alcaline osseuse, l'ostéocalcine et les propeptides d'extension du procollagène (Srivastava, 2005 et Vesper, 2005) :

- la phosphatase alcaline (PA ou ALPL) est une enzyme ubiquitaire. On distingue chez l'homme 6 isoenzymes : hépatique, intestinale, osseuse, rénale, placentaire et tumorale. Chez l'adulte présentant une fonction hépatique normale, environ 60-70% de l'activité sérique de la PA provient du foie, 30-40% provient de l'os et moins de 5% provient des intestins. Le taux circulant de la PA osseuse dépend de l'activité des ostéoblastes mais aussi de son élimination hépatique, ce qui a pour conséquence, en cas d'affection hépatique, une possible modification du taux de la PA osseuse circulante;
- l'ostéocalcine (OC) est une protéine de 5,7 kDa sécrétée par les ostéoblastes. Dans la circulation sanguine, l'ostéocalcine est rapidement dégradée et éliminée (1/3 de l'ostéocalcine circulante est représenté par la protéine entière et 2/3 représenté par des fragments de différentes tailles), ce qui limite son application clinique en tant que marqueur de la formation osseuse ;
- le collagène de type I est synthétisé par les ostéoblastes sous forme de procollagène. Ce précurseur contient deux propeptides aux extrémités N et C terminales, respectivement PINP (propeptide d'extension N-terminale) et PICP (propeptide d'extension C-terminale), qui sont clivés par des protéases lors de l'assemblage du procollagène en triple hélice et libérés dans la circulation. Le dosage sérique de ces propeptides permet donc d'évaluer la formation du collagène de type I. Cependant, ce dosage reflète la synthèse du collagène de type I non seulement au niveau de l'os mais aussi au niveau d'autres tissus.

La demande WO 2007/104724 décrit l'utilisation de marqueurs biologiques dans le suivi de l'ostéogénèse, en particulier pour le diagnostic de pathologies osseuses métaboliques, mais aussi pour le diagnostic de tumeurs osseuses. En ce qui concerne les tumeurs osseuses, la demande WO 2007/104724 enseigne l'intérêt de la pyruvate kinase M1, la chaine B de la L-lactate dehydrogénase, la triosephosphate isomerase 1, la chaine B de la créatine kinase, la protéine hsp90, la protéine ORP150 (pour « 150KDa oxygen-regulated protein »), la protéine pRb4 (Retinoblastoma-binding protein 4), l'alcaline phosphatase, la galectine-1, l'annexine, la tropomyosine 3 ou encore la kinase UMP-CMP, en tant que marqueurs biologiques. L'analyse de ces marqueurs biologiques nécessite cependant une étape de culture *in vitro* des cellules osseuses du sujet, ainsi que des étapes de purification du milieu de culture, ce qui limite leur utilité en clinique. Ainsi, les marqueurs de l'ostéogenèse actuellement utilisés ne sont pas entièrement satisfaisants. Il en résulte donc un besoin d'identifier de nouveaux marqueurs de l'ostéogenèse.

Les antigènes du complexe majeur d'histocompatibilité (CMH), se divisent en plusieurs classes : les antigènes de classe I (HLA-A, HLA-B et HLA-C) qui présentent 3 domaines globulaires ($\alpha$1, $\alpha$2 et $\alpha$3), et dont le domaine $\alpha$3 est associé à la $\beta$2 micro-globuline, les antigènes de classe II (HLA-DP, HLA-DQ et HLA-DR) et les antigènes de classe III (complément). Les antigènes de classe I comprennent, outre les antigènes précités, d'autres antigènes, dits antigènes de classe I non classiques (classe Ib) et notamment les antigènes HLA-E, HLA-F et HLA-G.

La séquence nucléotidique du gène HLA-G (gène HLA-6.0) a été décrite par Geraghty *et al.* (1987) : il comprend 4396 paires de bases et présente une organisation en intron/exon homologue à celle des gènes HLA-A, -B et -C. Ce gène comprend 8 exons, 7 introns et une extrémité 3' non traduite. Le gène HLA-G diffère des autres gènes du CMH de classe I, en ce que le codon de terminaison de la traduction en phase est localisé au niveau du deuxième codon de l'exon 6 ; en conséquence, la région cytoplasmique de la protéine codée par ce gène HLA-6.0 est plus courte que celle des régions cytoplasmiques des protéines HLA-A, -B et -C.

Ishitani et Geraghty (1992) ont montré que le transcrit primaire du gène HLA-G peut être épissé de plusieurs manières et produit au moins 3 ARNm matures distincts : le transcrit primaire d'HLA-G fournit une copie complète (G1) de 1200 pb, un fragment de 900 pb (G2) et un fragment de 600 pb (G3). Le transcrit G1 ne comprend pas l'exon 7 et correspond à la séquence décrite par Ellis *et al.* (1990), c'est-à-dire qu'il code une protéine qui comprend une séquence signal, trois domaines globulaires extracellulaires ($\alpha$1, $\alpha$2 et $\alpha$3), un domaine transmembranaire et un domaine intra-cytoplasmique. L'ARNm G2 ne comprend pas l'exon 3 (codant le domaine $\alpha$2), et code une isoforme dans laquelle les domaines $\alpha$1 et $\alpha$3 sont directement joints. L'ARNm G3 ne contient ni l'exon 3, ni l'exon 4 (codant le domaine $\alpha$3) ; ce transcrit code donc une isoforme dans laquelle le domaine $\alpha$1 et le domaine transmembranaire sont directement joints. L'épissage qui prévaut pour l'obtention de l'antigène HLA-G2 entraîne la jonction d'une adénine (A) (provenant du domaine codant $\alpha$1) avec une séquence adénine-cytosine (AC) (issue du domaine codant $\alpha$3), ce qui entraîne la création d'un codon AAC (asparagine) à la place du codon GAC (acide aspartique), présent en position 5' de la séquence codant le domaine $\alpha$3 dans HLA-G1. L'épissage généré pour l'obtention de HLA-G3 n'entraîne pas la formation d'un nouveau codon dans la zone d'épissage.

Certains des Inventeurs ont montré l'existence d'autres formes épissées d'ARNm d'HLA-G : le transcrit *HLA-G4,* qui n'inclut pas l'exon 4 ; le transcrit *HLA-G5,* qui inclut l'intron 4, entre les exons 4 et 5, provoquant ainsi une modification du cadre de lecture, lors de la traduction de ce transcrit et en particulier l'apparition d'un codon stop après l'acide aminé 21 de l'intron 4 ; le transcrit *HLA-G6,* possédant l'intron 4, mais ayant perdu l'exon 3 ; et le transcrit *HLA-G7* qui inclut l'intron 2, provoquant ainsi une modification du cadre de lecture, lors de la traduction de ce transcrit et l'apparition d'un codon stop après l'acide aminé 2 de l'intron 2 (Kirszenbaum *et al.,* 1994 et 1995 ; Moreau *et al.,* 1995 ; Demande Européenne EP 0 677 582).

Il existe donc au moins 7 ARNm *HLA-G* différents qui codent 7 isoformes d'HLA-G dont 4 membranaires (HLA-G1, -G2, -G3 et -G4) et 3 solubles (HLA-G5, -G6 et -G7, qui ne comprennent pas de domaine transmembranaire) (voir pour revue Carosella *et al.,* 2008a).

La séquence nucléotidique du gène *HLA-G* (gène *HLA-6.0*) et son organisation en exon/intron, ainsi que les séquences d'acides aminés des différentes isoformes d'HLA-G sont bien connues de l'Homme du métier. Elles ont notamment été décrites par Geraghty *et al.* (1987) et par Carosella *et al.* (2008a) citées ci-dessus.

L'expression protéique d'HLA-G est normalement restreinte aux trophoblastes (à l'interface materno-foetale), au thymus, à la cornée, aux précurseurs endothéliaux et érythroblastiques et aux cellules souches mésenchymateuses (Carosella *et al.,* 2008a). Toutefois, les ARNm *HLA-G* sont détectés dans quasiment toutes les cellules de l'organisme à un taux basal qui peut être amplifié et dont la traduction en protéine est induite sous l'effet d'agents déméthylants de l'ADN, de cytokines telles que les interférons (IFN), de facteurs de stress ou d'hypoxie (Carosella *et al.,* 2008b). Ainsi, dans des conditions particulières telles que la greffe d'un tissu, le développement de certaines tumeurs ou une réponse inflammatoire, la protéine HLA-G pourra être exprimée dans des tissus qui ne l'expriment pas en condition normale.

Dans le sang, on retrouve aussi bien les isoformes solubles que les isoformes membranaires détachées de la membrane, telles que HLA-G1 (qui est aussi dénommée HLA-G1s, pour « HLA-G1 shedding »).

Plusieurs propriétés biologiques d'HLA-G ont été identifiées : inhibition de la cytolyse médiée par les cellules NK et les CTL, inhibition de la réponse T alloproliférative, induction d'une apoptose dans les cellules T et les cellules NK CD8[+], et action antiproliférative sur les cellules B du système immunitaire (Carosella *et al.,* 2008a et 2008b).

Certains des Inventeurs ont récemment montré que les cellules souches mésenchymateuses (CSM) en culture secrètent HLA-G5, exerçant ainsi une activité immunosuppressive vis-à-vis de la réponse immunitaire T et NK (« *Natural Killer* ») (Selmani *et al.,* 2008).

Les cellules souches mésenchymateuses dérivées de la moelle osseuse adulte sont des cellules multipotentes, précurseurs des ostéoblastes, des chondroblastes et des adipocytes (Friedenstein *et al.,* 1976 ; Pittenger *et al.,* 1999).

Dans le cadre de leurs travaux, les Inventeurs ont cherché si les ostéoblastes, chondroblastes et adipocytes obtenus en culture à partir de CSM pouvaient, eux aussi, exprimer HLA-G. De manière surprenante, les Inventeurs ont montré, par la méthode ELISA, que seuls les ostéoblastes exprimaient les formes solubles HLA-G. Ils ont aussi montré, par la technique de RT-PCR, que les ostéoblastes exprimaient les ARNm *HLA-G1, -G2, -G3, -G4 et -G5.* En outre, les Inventeurs ont observé chez l'Homme, *in vivo,* qu'HLA-G n'est exprimée par les ostéoblastes normaux ou pathologiques (notamment tumoraux) que lors de la formation osseuse (ostéogenèse).

Sur la base de ces résultats montrant une expression d'HLA-G par les ostéoblastes lors de la formation osseuse, l'évaluation d'une ostéogenèse chez un sujet, que ce soit au cours d'une reconstruction osseuse post-fracture ou dans le cadre du suivi de l'évolution d'une tumeur osseuse, peut donc être effectuée par dosage ou détermination de l'expression par les ostéoblastes d'au moins une isoforme d'HLA-G : une augmentation de la concentration ou de l'expression d'au moins une isoforme d'HLA-G étant l'indication d'une ostéogenèse.

L'objet de la présente invention est défini par les revendications 1 à 6.

La présente invention concerne une méthode *in vitro* de suivi de l'évolution d'une tumeur osseuse chez un sujet, à partir d'échantillons biologiques comprenant des ostéoblastes, obtenus par biopsie osseuse de ladite tumeur à un temps t0 et à un temps t1, caractérisée en ce qu'elle comprend une étape de détermination de la concentration ou de détermination quantitative de l'expression des isoformes HLA-G1 et HLA-G5 dans lesdits échantillons biologiques,

- une augmentation de la concentration ou du niveau d'expression d'au moins une desdites isoformes d'HLA-G entre les temps t0 et t1 étant l'indication d'une progression de ladite tumeur osseuse chez ledit sujet,
- une diminution de la concentration ou du niveau d'expression d'au moins une desdites isoformes d'HLA-G entre les temps t0 et t1 étant l'indication d'une rémission de ladite tumeur osseuse chez ledit sujet.

La présente description a en conséquence pour objet une méthode *in vitro* de suivi de la reconstruction osseuse chez un sujet, chez lequel a été diagnostiquée une fracture osseuse, laquelle méthode comprend les étapes suivantes :

a) mesure de la concentration d'au moins une isoforme d'HLA-G dans un échantillon de liquide biologique dudit sujet,
b) comparaison de la concentration de ou des isoformes d'HLA-G mesurée à l'étape a) avec une concentration de référence de cette ou de ces isoformes d'HLA-G dans ledit liquide biologique chez les sujets sains,

une concentration d'au moins une isoforme d'HLA-G, dans ledit échantillon de liquide biologique dudit sujet, supérieure à ladite concentration de référence étant l'indication d'une reconstruction osseuse (ostéogenèse) chez ledit sujet.

On entend par « fracture osseuse » une rupture de la continuité d'un os. Ce peut être notamment une fissure osseuse (fracture sans déplacement de l'os) ou une fracture comminutive (comprenant plusieurs fragments osseux ; fracture multi-esquilleuse). Une fracture osseuse peut être diagnostiquée, par exemple, par radiographie, scintigraphie ou tomodensitométrie.

On entend par « sujet » au sens de la présente description, un mammifère, de préférence un humain.

Dans un mode de réalisation, l'invention concerne une méthode telle que décrite précédemment, caractérisée en ce que ledit sujet est un humain.

On entend par « sujets sains » au sens de la présente description, les sujets qui ne présentent pas de lésion osseuse, c'est-à-dire une fracture osseuse.

On entend par « liquide biologique » au sens de la présente description, le sang et ses dérivés (tels que le plasma et le sérum), ainsi que le liquide synovial, de préférence le sang.

On entend par « isoforme d'HLA-G » au sens de la présente description, une isoforme d'HLA-G choisie parmi les isoformes d'HLA-G membranaires (HLA-G1, -G2, -G3 et -G4) et solubles (HLA-G5, -G6 et -G7).

Bien entendu, lorsque l'on mesure la concentration d'une isoforme membranaire d'HLA-G dans un liquide biologique, cela signifie que cette isoforme est détachée de la membrane cellulaire (par protéolyse par exemple) et se retrouve dans ledit liquide biologique.

Selon un mode de mise en oeuvre préféré de ladite méthode de suivi de la reconstruction osseuse, on mesure la concentration d'au moins une isoforme d'HLA-G choisie parmi HLA-G1, HLA-G5, HLA-G6 et HLA-G7.

Selon un autre mode de mise en oeuvre préféré de ladite méthode de suivi de la reconstruction osseuse, on mesure la concentration de deux isoformes d'HLA-G différentes, de préférence HLA-G1 et HLA-G5, ou HLA-G5 et HLA-G6.

Selon un autre mode de mise en oeuvre préféré de ladite méthode de suivi de la reconstruction osseuse, on mesure, à partir d'un échantillon de sang, la concentration plasmatique ou sérique d'au moins une isoforme d'HLA-G telle que définie ci-dessus, de préférence choisie parmi HLA-G1, HLA-G5, HLA-G6 et HLA-G7, ou de préférence encore de deux isoformes d'HLA-G différentes, telles que HLA-G1 et HLA-G5, et HLA-G5 et HLA-G6.

La mesure de la concentration plasmatique d'une isoforme d'HLA-G à partir d'un échantillon de sang est bien connue de l'Homme du métier. Elle peut s'effectuer en mettant en oeuvre une méthode immunologique appropriée (e.g., ELISA, RIA, Immunofluorescence, Immunohistochimie) au moyen d'au moins un anticorps spécifique de ladite isoforme d'HLA-G.

Dans un mode de réalisation, l'invention concerne une méthode telle que décrite précédemment, caractérisée en ce que l'on détermine ladite concentration ou ladite expression d'au moins une desdites isoformes d'HLA-G dans lesdits échantillons biologiques par une méthode immunologique appropriée.

On entend par « anticorps » au sens de la présente description, un anticorps polyclonal ou monoclonal ; humain ou non humain, par exemple murin, humanisé, chimérique ; recombinant ou synthétique ; ou un fragment d'anticorps (par exemple les fragments Fab'2 ou Fab) comprenant un domaine de l'anticorps initial reconnaissant l'antigène cible dudit anticorps initial.

De nombreux anticorps, monoclonaux ou polyclonaux, spécifiques d'une ou plusieurs isoformes d'HLA-G sont connus de l'Homme du métier. A titre d'exemple d'anticorps monoclonaux anti-HLA-G humain disponibles commercialement, on peut citer l'anticorps anti-HLA-G (reconnaissant toutes les isoformes d'HLA-G) obtenu à partir du clone 4H84 (McMaster *et al.,* 1998), l'anticorps anti-HLA-G1, -G2, -G5 et G6 (c'est-à-dire reconnaissant les isoformes HLA-G1, -G2, -G5 et G6) obtenu à partir du clone MEM-G/4 (aussi dénommé MEM-G/04 ; Menier *et al.,* 2003), les anticorps anti-HLA-G1 et anti-HLA-G5 (c'est-à-dire reconnaissant les isoformes HLA-G1 et -G5) obtenus à partir des clones MEM-G/9 (aussi dénommé MEM-G/09 ; Menier *et al.,* 2003) ou 87G (Rebmann *et al.,* 1999 ; Hackmon *et al.,* 2004), et l'anticorps anti-HLA-G5 et anti-HLA-G6 (c'est-à-dire reconnaissant les isoformes HLA-G5 et -G6) obtenu à partir du clone 5A6G7 (Le Rond *et al.,* 2004).

La concentration de référence d'une isoforme d'HLA-G dans un liquide biologique tel que défini ci-dessus est fonction non seulement de l'isoforme d'HLA-G donnée mais aussi de la méthode utilisée pour la mesure de la concentration.

La concentration plasmatique de référence des isoformes HLA-G1 (HLA-G1s) et HLA-G5 chez les personnes saines, c'est-à-dire ne présentant pas de fracture osseuse, mesurée par ELISA en utilisant l'anticorps monoclonal obtenu à partir du clone MEM-G/9, est inférieure à 20 ng/mL (Ugurel *et al.,* 2001 ; Le Rond *et al.,* 2006 ; Naji *et al.,* 2007).

Ainsi, selon une disposition particulière de ce mode de mise en oeuvre, une concentration plasmatique des isoformes HLA-G1 (HLA-G1s) et HLA-G5 supérieure à 20 ng/mL, de préférence supérieure à 50 ng/ml, de préférence encore supérieure à 100 ng/mL, mesurée par une méthode immunologique appropriée, de préférence par ELISA, en utilisant un anticorps à la fois anti-HLA-G1 et anti-HLA-G5 (par exemple l'anticorps monoclonal obtenu à partir du clone MEM-G/9), à partir d'un échantillon de sang dudit sujet humain chez lequel a été diagnostiquée une fracture osseuse, est l'indication d'une reconstruction osseuse chez ledit sujet.

La concentration plasmatique de référence des isoformes HLA-G5 et HLA-G6 chez lesdites personnes saines, mesurée par ELISA en utilisant l'anticorps monoclonal obtenu à partir du clone 5A6G7, est inférieure à 10 ng/mL (Le Rond *et al.,* 2006 ; Naji *et al.,* 2007).

Ainsi, selon une autre disposition particulière de ce mode de mise en oeuvre, une concentration plasmatique des isoformes HLA-G5 et HLA-G6 supérieure à 10 ng/mL, de préférence supérieure à 50 ng/ml, de préférence encore supérieure à 100 ng/mL, mesurée par une méthode immunologique appropriée, de préférence par ELISA, en utilisant un anticorps à la fois anti-HLA-G5 et anti-HLA-G6 (par exemple l'anticorps monoclonal obtenu à partir du clone 5A6G7), à partir d'un échantillon de sang dudit sujet humain chez lequel a été diagnostiquée une fracture osseuse, est l'indication d'une reconstruction osseuse chez ledit sujet.

La concentration sérique de référence des isoformes HLA-G1 (HLA-G1s) et HLA-G5 chez lesdites personnes saines, mesurée par ELISA en utilisant l'anticorps monoclonal obtenu à partir du clone MEM-G/9, est d'environ 20 ng/mL (Rouas-Freiss *et al.,* 2005).

Ainsi, selon une autre disposition particulière de ce mode de mise en oeuvre, une concentration plasmatique des isoformes HLA-G1 (HLA-G1s) et HLA-G5 supérieure à 25 ng/mL, de préférence supérieure à 50 ng/ml, de préférence encore supérieure à 100 ng/mL, mesurée par une méthode immunologique appropriée, de préférence par ELISA, en utilisant un anticorps à la fois anti-HLA-G1 et anti-HLA-G5 (par exemple l'anticorps monoclonal obtenu à partir du clone MEM-G/9), à partir d'un échantillon de sang dudit sujet humain chez lequel a été diagnostiquée une fracture osseuse, est l'indication d'une reconstruction osseuse chez ledit sujet.

La présente description a aussi pour objet une méthode *in vitro* de suivi de l'évolution (progression ou rémission) d'une tumeur osseuse chez un sujet (chez lequel à été diagnostiquée une tumeur osseuse), à partir d'échantillons biologiques dudit sujet obtenus à un temps t0 et à un temps t1, laquelle méthode comprend une étape de détermination de la concentration ou de détermination quantitative de l'expression d'au moins une isoforme d'HLA-G dans lesdits échantillons biologiques,

- une augmentation de la concentration ou du niveau d'expression d'au moins une isoforme d'HLA-G entre les temps t0 et t1 étant l'indication d'une progression de ladite tumeur osseuse chez ledit sujet,
- une diminution de la concentration ou du niveau d'expression d'au moins une isoforme d'HLA-G entre les temps t0 et t1 étant l'indication d'une rémission (ou régression) de ladite tumeur osseuse chez ledit sujet.

On entend par « échantillon biologique » au sens de la présente description, un échantillon de liquide biologique tel que défini ci-dessus ou un échantillon biologique comprenant des ostéoblastes qui a été obtenu par biopsie osseuse de la tumeur.

Dans un mode de réalisation, l'invention concerne une méthode telle que décrite précédemment, caractérisée en ce que l'on détermine quantitativement l'expression par les ostéoblastes d'au moins une desdites isoformes d'HLA-G, à partir d'échantillons biologiques comprenant des ostéoblastes.

La biopsie peut être un prélèvement de toute partie du squelette, telle que la colonne vertébrale, le bassin, le fémur, le tibia, l'humérus, l'omoplate, et le crâne.

Dans un mode de réalisation, l'invention concerne une méthode telle que décrite précédemment, caractérisée en ce que la tumeur osseuse est choisie dans le groupe constitué par l'ostéosarcome, l'ostéoblastome, le sarcome d'Ewing et les tumeurs à cellules géantes.

Selon un mode de mise en oeuvre préféré de cette méthode d'évaluation *in vitro* d'une tumeur osseuse, on détermine la concentration plasmatique ou sérique, par exemple par une méthode immunologique appropriée telle que définie ci-dessus, d'au moins une isoforme d'HLA-G telle que définie ci-dessus, à partir d'échantillons de sang dudit sujet humain.

Selon une disposition avantageuse de ce mode de mise en oeuvre, on détermine la concentration de deux isoformes d'HLA-G différentes, de préférence HLA-G1 et HLA-G5, ou HLA-G5 et HLA-G6.

Selon un autre mode de mise en oeuvre préféré de cette méthode d'évaluation *in vitro* d'une tumeur osseuse, on détermine quantitativement l'expression par les ostéoblastes d'au moins une isoforme d'HLA-G telle que définie ci-dessus, à partir d'échantillons biologiques comprenant des ostéoblastes, lesquels échantillons ont été obtenus par biopsie osseuse de ladite tumeur.

Selon une disposition avantageuse de ce mode de mise en oeuvre, l'expression d'au moins une isoforme d'HLA-G par les ostéoblastes est déterminée *in vitro* par une méthode immunologique telle que définie ci-dessus, de préférence une méthode immunohistochimique appropriée, au moyen d'au moins un anticorps spécifique de ladite isoforme d'HLA-G tel que défini ci-dessus.

Selon une autre disposition avantageuse de ce mode de mise en oeuvre, l'expression d'au moins une isoforme d'HLA-G par les ostéoblastes est déterminée *in vitro* par détection des ARNm codant au moins une isoforme d'HLA-G.

Dans un mode de réalisation, l'invention concerne une méthode telle que décrite précédemment, caractérisée en ce que l'on détermine quantitativement l'expression par les ostéoblastes d'au moins une desdites isoformes d'HLA-G par la détection des ARNm codant ladite isoforme d'HLA-G.

La détection des ARNm peut être effectuée par hybridation, au moyen de sondes nucléotidiques spécifiques desdits ARNm (fixées par exemple sur une biopuce), ou par amplification (par exemple par RT-PCR), au moyen d'amorces nucléotidiques spécifiques desdits ARNm. A titre d'exemple d'amorces aptes à amplifier les ARNm *HLA-G* on peut citer le couple d'amorces constitué des séquences nucléotidiques SEQ ID NO : 15 et 16, et ceux décrits par Le Discorde *et al.,* 2005.

La présente description a aussi pour objet une méthode de criblage *in vitro* d'un agent qui module (augmente ou diminue) l'ostéogenèse, laquelle méthode comprend les étapes suivantes :

a) détermination quantitative de l'expression d'au moins une isoforme d'HLA-G par des ostéoblastes ;
b) mise en contact desdits ostéoblastes avec un agent à tester ; puis
c) détermination quantitative de l'expression de ladite au moins une isoforme d'HLA-G par lesdits ostéoblastes,

une différence du niveau d'expression par lesdits ostéoblastes d'au moins une isoforme d'HLA-G, entre les étapes a) et c), étant l'indication que ledit agent module l'ostéogenèse.

Une augmentation du niveau d'expression, par lesdits ostéoblastes, d'au moins une isoforme d'HLA-G, entre les étapes a) et c) ci-dessus, est l'indication que ledit agent induit un processus d'ostéogenèse.

Une diminution du niveau d'expression, par lesdits ostéoblastes, d'au moins une isoforme d'HLA-G, entre les étapes a) et c) ci-dessus, est l'indication que ledit agent réprime un processus d'ostéogenèse.

La détermination quantitative de l'expression d'au moins une isoforme d'HLA-G par les ostéoblastes peut être effectuée par une méthode telle que décrite ci-dessus, c'est-à-dire par une méthode immunologique ou par détection des ARNm codant les isoformes d'HLA-G.

Selon un mode de mise en oeuvre préféré de ladite méthode de criblage, les ostéoblastes sont d'origine humaine.

Selon un autre mode de mise en oeuvre préféré de ladite méthode de criblage, on détermine l'expression d'HLA-G1 et HLA-G5, ou d'HLA-G5 et HLA-G6.

La présente description a aussi pour objet une isoforme d'HLA-G ou une molécule d'acide nucléique (par exemple un ARNm) codant une isoforme d'HLA-G, isolée d'un sujet, de préférence un humain, pour utilisation comme marqueur, de préférence comme marqueur sanguin, de l'ostéogenèse chez ledit sujet.

Selon un mode de réalisation préféré de l'description, ladite isoforme d'HLA-G est choisie parmi HLA-G1, HLA-G5 et HLA-G6.

Outre les dispositions qui précèdent, l'description comprend encore d'autres dispositions, qui ressortiront de la description qui va suivre, qui se réfère à des exemples de mise en oeuvre de la méthode objet de la présente description ainsi qu'aux dessins annexés, dans lesquels :

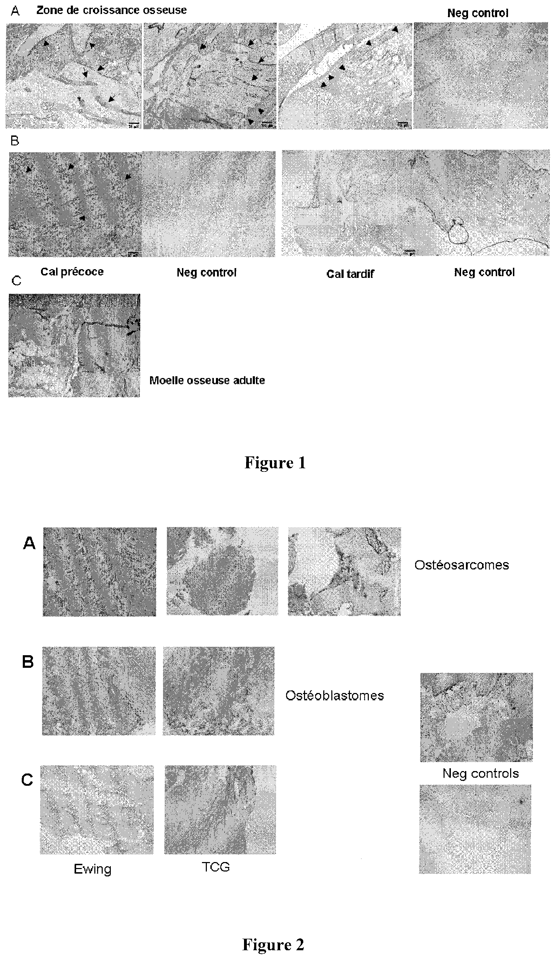

- la Figure 1 montre l'expression d'HLA-G par les ostéoblastes des zones de croissance osseuse chez le nouveau-né (**A**) et de cals post-fracture chez l'adulte (**B**). Des coupes fines de doigts surnuméraires et de cals précoces et tardifs ont été incubées avec un anticorps anti-HLA-G5 et anti-HLA-G6 (clone 5A6G7) puis avec un anticorps secondaire anti-souris conjugué à la peroxydase. Les lames ont ensuite été observées au microscope photonique. Au niveau des zones de croissance osseuse, les ostéoblastes situés le long des trabécules osseux ainsi que du périoste sont marqués par l'anticorps anti-HLA-G5/-G6 (voir flèches noires). Au niveau des cals osseux, les ostéoblastes condensés au niveau de trabécules osseux néo-formés sont également marqués par l'anticorps anti-HLA-G5/-G6. (**C**) : aucune cellule HLA-G+ n'a pu être observée dans une moelle osseuse adulte non pathologique ;
- la Figure 2 montre la détection d'HLA-G au niveau de tumeurs osseuses. Des coupes fines de biopsies de tumeurs de l'os provenant d'ostéosarcomes (**A**), d'ostéoblastomes (**B**), de sarcomes d'Ewing et de tumeurs à cellules géantes (**C**) ont été incubées avec un anticorps anti-HLA-G5/-G6 (clone 5A6G7). Les ostéoblastes tumoraux d'ostéosarcomes et d'ostéoblastomes sont marqués (**A** et **B**) alors que seuls les ostéoblastes normaux du microenvironnement tumoral des sarcomes d'Ewing ou de tumeurs à cellules géantes sont positifs pour l'expression d'HLA-G5/-G6 (C) ;

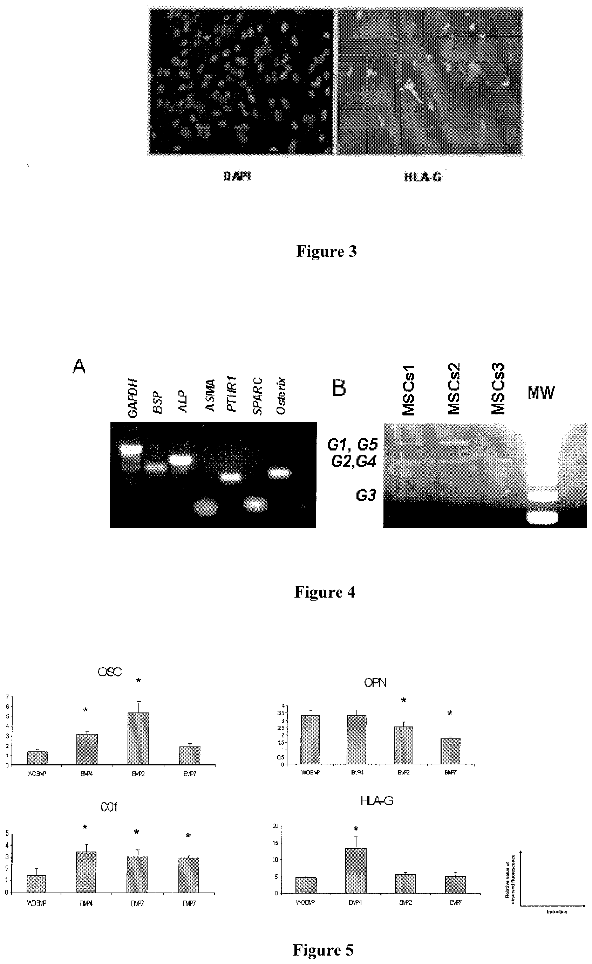

- la Figure 3 montre la détection d'HLA-G dans la lignée SaOs2 par immunofluorescence *in situ.* Les cellules de la lignée d'ostéosarcome SaOs2 ont été cultivées en chambre de culture contenant des puits, puis fixées et incubées avec un anticorps anti-HLA-G1 et anti-HLA-G5 (clone 87G) conjugué au FITC. Les noyaux des cellules ont été marqués au DAPI. Grossissement X400 ;
- la Figure 4 montre l'expression de l'ARNm d'HLA-G par des ostéoblastes obtenus à partir de CSMs. Les ostéoblastes ont été lysées et l'ARNm récupéré. Après transcription inverse, les ADNc ont été amplifiés par PCR. (**A**) : les transcrits (ARNm *BSP, PA, PTHR1, SPARC, Osterix* et *ASMA*) caractéristiques des ostéoblastes ont été recherchés sur les cellules cultivées dans le milieu ostéogénique. L'ARNm *GAPDH* (glycéraldéhyde-3-phosphate déshydrogénase ; gène constitutif) a été utilisé en tant que contrôle. (**B**) : l'expression des différentes formes d'*HLA-G* (*G1, G2, G3, G4* et *G5*) dans 3 cultures d'ostéoblastes différentes obtenues à partir de CSMs (MSC1, MSC2, MSC3) a été recherchée ;
- la Figure 5 montre l'expression d'*HLA-G* par des ostéoblastes obtenus à partir de CSMs par immunofluorescence *in situ.* Les CSMs ont été cultivées en chambre de culture contenant des puits et à confluence, et induites pour se différencier en ostéoblastes. Les inducteurs utilisés étaient : BMP-2, BMP-4 et BMP-7. Des CSMs cultivées dans un milieu non inducteur (milieu d'expansion) ont été utilisées comme contrôle négatif (WOBMP). Après 10 jours de culture, les ostéoblastes ont été fixés et perméabilisés avant d'être incubés avec les anticorps anti-collagène 1 (CO1), anti-ostépontine (OPN), anti-ostéocalcine (OSC) et anti-HLA-G1/- G5 (clone 87G). Les noyaux des cellules ont été marqués par l'ajout de DAPI dans le milieu réactionnel. La fluorescence a ensuite été détectée au microscope à épifluorescence. Les fluorescences émises par la présence des anticorps a été rapportée à celle émise par le DAPI pour tenir compte d'un taux d'expression des molécules recherchées sur le nombre de cellules présentes dans les puits (« *relative value of observed fluorescence* ») ;

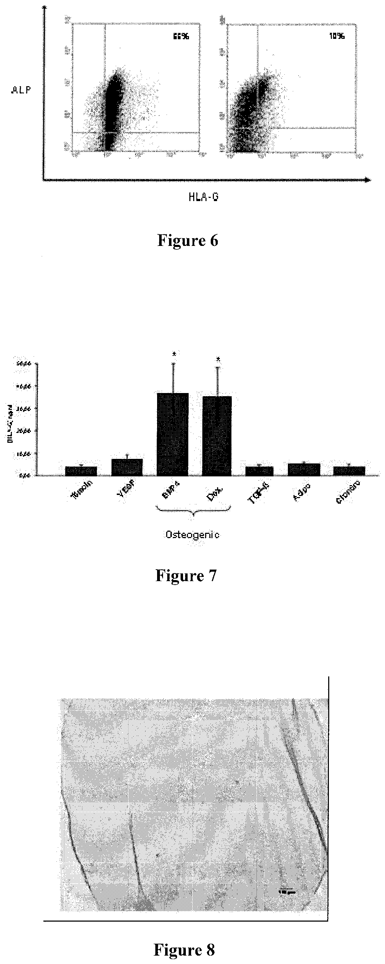

- la Figure 6 montre l'expression d'HLA-G par cytométrie en flux au cours de l'ostéogenèse. Les CSMs ont été cultivées dans un milieu ostéogénique et les expressions conjointes d'HLA-G5/-G6 et de la phosphatase alcaline (PA) ont été suivies par cytométrie en flux à la $2^{\text{ème}}$ (**A**) et $4^{\text{ème}}$ (**B**) semaine de culture. Le nombre de cellules HLA-G+ diminue de 66% (semaine 2) à 10% (semaine 4). La Figure est représentative de 4 expériences indépendantes ; les valeurs données sont les moyennes observées sur l'ensemble des expériences ;
- la Figure 7 montre le dosage d'*HLA-G* soluble dans les surnageants de cultures d'ostéoblastes obtenus à partir de CSMs. Les CSMs ont été cultivées dans des milieux inducteurs de différenciation ostéogénique (BMP4, Dexaméthasone), chondrogénique (Chondro, TGF-β), adipogénique (Adipo) et angiogénique (VEGF) ou en l'absence de toute induction (témoin). Après 4 jours d'incubation, des aliquots des surnageants ont été prélevés et les isoformes HLA-G5 et -G6 y ont été dosées par ELISA. Les résultats sont rapportés en ng/mL ;
- la Figure 8 montre la recherche de l'expression d'*HLA-G* dans les chondrocytes après culture en micro-masse. Les CSMs ont été cultivées dans des conditions de micro-masse dans un milieu chondrogénique. Après 4 semaines de culture, les micro-masses ont été récupérées, fixées et incluses en paraffine. Les coupes fines réhydratées ont été incubées soit avec un anticorps anti-HLA-G (clone 5A6G7) soit avec un anticorps contrôle (contrôle isotypique). Après lavage, les coupes ont été colorées à l'hématoxyline / éosine puis observées au microscope photonique ;

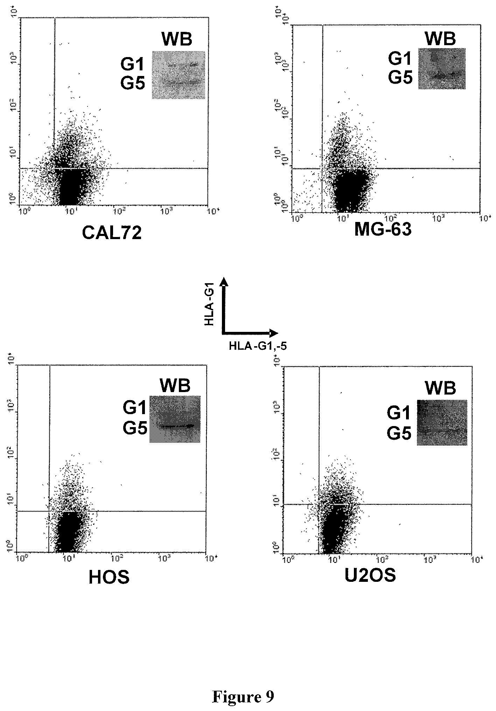

- la Figure 9 montre l'expression d'HLA-G1 et d'HLA-G5 par les lignées ostéoblastiques humaines CAL72, MG-63, HOS et U2OS dérivées d'ostéosarcomes (OS). WB = *Western Blot* ;

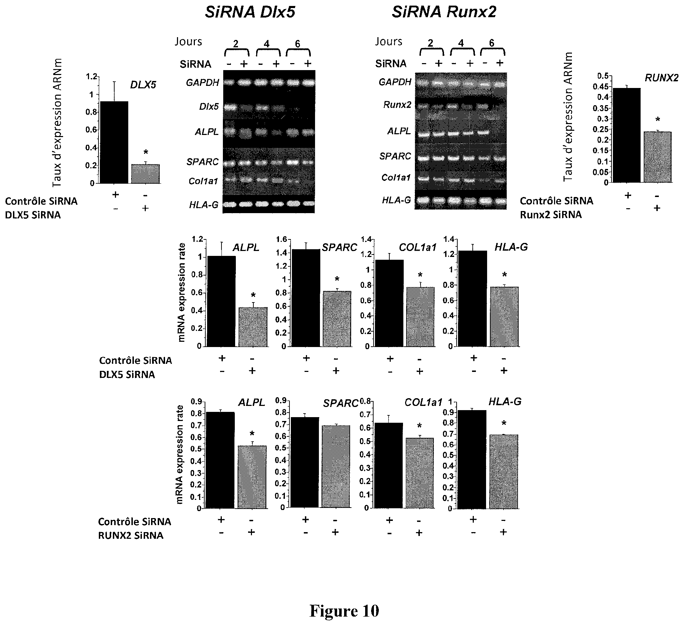

- la Figure 10 montre l'effet de l'inhibition des gènes *DLX5* ou *RUNX2* sur l'expression de *GAPDH, ALPL, SAPRC, COL1α1, HLA-G1 et HLA-G5* dans des cellules souches mésenchymateuses de moelles osseuse humaines induites pour se différencier en ostéoblastes. L'analyse a été effectuée par RT-PCR.

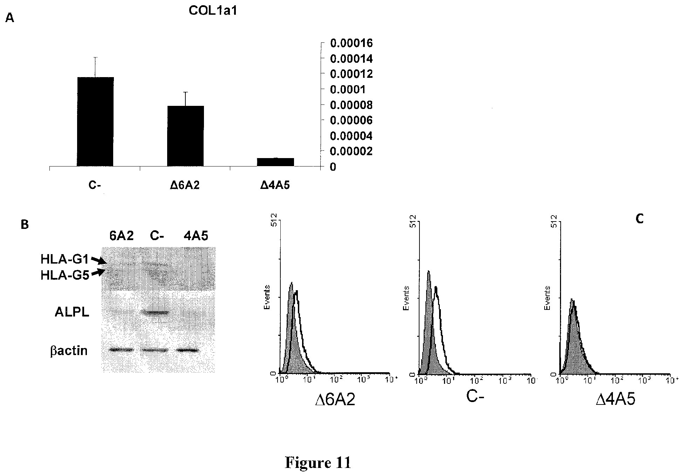

- la Figure 11 montre l'expression d'HLA-G1 et G-5, COL1α1 et ALPL par les lignées ostéoblastiques d'origine humaine Δ4A5 et Δ6A2, sur-exprimant RUNX2. **A** : analyse par qPCR. **B** : analyse par Western blot ; les isoformes HLA-G1 et HLA-G5 ont été détectées, l'isoforme β de l'actine a servi de contrôle positif interne. **C** : analyse par cytométrie en flux ; l'expression de HLA-G1 et -G5 est montrée par l'histogramme à ligne épaisse alors que le seuil de négativité est donné par l'histogramme du contrôle isotypique (en gris).

## EXEMPLE 1: MISE EN EVIDENCE DE L'EXPRESSION D'HLA-G PAR LES OSTEOBLASTES

### 1) Matériel et Méthode

*Cellules et cultures cellulaires :*

Les échantillons de moelle osseuse (MO) humaine d'adultes ont été collectés sur des volontaires sains subissant une chirurgie orthopédique. Ces prélèvements ont été réalisés selon les recommandations du comité d'éthique du CHU Trousseau à Tours, France. Les cellules mononucléées (CMN) ont été ensemencées dans du milieu alpha-MEM (MEM) supplémenté avec 10% de sérum de veau foetal (SVF) (Perbio Hyclone; Logan) et 1% de pénicilline/streptomycine (InVitrogen Ltd; Paisley, UK). Au 14ème jour, à 80% de confluence, les cellules souches mésenchymateuses (CSMs) ont été dupliquées et amplifiées. Toutes les expériences ont été réalisées avec au moins 3 donneurs différents.

La lignée d'ostéosarcome SaOs2 (ECECC, Salisbury, UK) a été cultivée dans les mêmes conditions que pour l'expansion des CSMs.

*Induction ostéoblastiaue et chondroblastique :*

Les CSMs ont été induites à se différencier en ostéoblastes et chondrocytes en suivant les protocoles décrits par Pittenger *et al.,* 1999.

Brièvement, pour induire l'ostéogenèse, le milieu est composé de DMEM 4,5g/L de glucose, de $NaH_2PO_4$ (InVitrogen) 3 mM, d'acide ascorbique (Sigma) 25 mg/L et de dexaméthasone (InVitrogen) $10^{-7}$ M. Ce milieu permet la différenciation en ostéoblastes des cellules après 14 jours de culture.

Pour induire la chondrogenèse, les cellules ont été centrifugées et cultivées en micro-masse, c'est à dire en culot directement sans remise en suspension. Le milieu de différenciation utilisé est composé de DMEM 3M glucose (InVitrogen), de sodium pyruvate (Sigma, Saint Quentin Fallavier, France) 2 mM, d'acide ascorbique 2 phosphate (Sigma) 0,17 mM, de déxaméthasone (InVitrogen) $10^{-7}$ M, de proline (Sigma) 0,35 mM et un complément d'insuline transférine et sélénium (ITS) 1x (Sigma). Du « *Transforming Growth Factor beta type 1* » (TGFβ1) (AbCys) a été ajouté à une concentration de 10 ng/mL lors de chaque renouvellement de milieu. Ce milieu permet la différenciation chondrogénique après 21 jours de culture.

*Analyses par cytométrie en flux :*

Pour la détection membranaire et intracytoplasmique d'un antigène, 200 000 cellules ont été marquées par un anticorps monoclonal spécifique couplé à un fluorochrome. Le marquage membranaire a été obtenu après 30 minutes d'incubation à 4°C à l'obscurité. Les cellules ont ensuite été rincées avec du « *Phosphate Buffered Saline* » (PBS), puis fixées au CellFIX® (Becton Dickinson, Erembodegem, Belgique). Pour le marquage intracellulaire, les cellules ont été fixées et perméabilisées à l'aide du kit CytoFix/CytoPerm. Les cellules ont ensuite été passées au cytomètre en flux (FACS Calibur®, Becton Dickinson), équipé d'un laser Argon, émettant une longueur d'onde de 488 nm. Les données ont été analysées à l'aide du logiciel CellQuest 3.1® (Becton Dickinson) ; les résultats sont exprimés en rapport de moyenne d'intensité de fluorescence (RMFI) du signal détecté sur celui du bruit de fond. Les anticorps monoclonaux (mAb) suivants ont été utilisés : mAb anti-HLA-G1/-G5 (clone 87G) conjugué à l'Alexa 488 (Exbio, Vestec, Czech Republic), mAb anti-HLA-G5/-G6 (clone 5A6G7, Exbio) et le mAb anti-phosphatase alcaline (PA, désignée par ALP pour « *alkaline phosphatase* ») conjuguée à la phycoérythrine (PE).

*Analyses par ELISA (Enzyme Linked Immunosorbent Assay) :*

Les concentrations d'HLA-G solubles contenues dans les surnageants filtrés de cultures de CSMs après inductions ostéoblastiques, chondrocytaires, adipocytaires et vasculaires ont été mesurées. Le surnageant de cultures de cellules de la lignée M8-HLA-G5 a été utilisé comme contrôle positif (Le Rond *et al.,* 2006). La méthode ELISA utilisée comprend l'utilisation de l'anticorps anti-HLA-G5/-G6 (clone 5A6G7, Exbio) comme anticorps de capture et le pan-HLA classe I W6/32 comme anticorps de détection de molécules HLA de classe la (Betts *et al.,* 2003) (Rebmann *et al.,* 2005).

*Analyses par immunofluorescence in situ :*

Les cellules sélectionnées de 3 tumeurs fraîches ont été ensemencées dans des chambres de culture contenant des puits de 1 cm$^2$ de surface (Labteks®, Nunc International, Rochester, New York, USA) à 10 000 cellules/puits. Après 48 heures de culture en milieu de prolifération, elles ont été fixées 10 minutes avec du formaldéhyde (Sigma) à 3,7% ou au méthanol pur (InVitrogen). Une étape de perméabilisation avec une solution de PBS, 0,5% de SVF et 0,2% de Tween 20 (BioRad, Hercules, California, USA), pendant 30 minutes à température ambiante, a été nécessaire pour identifier les protéines intra-cellulaires. Les cellules ont ensuite été incubées successivement avec les anticorps primaires monoclonaux, pendant 1 heure à 4°C et les anticorps secondaires, reconnaissant les anticorps primaires, conjugués à un fluorochrome de type Alexa 488 ou 594 (InVitrogen). Après rinçage, un milieu de montage contenant du 4,6-diaminido-2-phénylindol (DAPI) (Vector Cliniscience, Montrouge, France) a été ajouté, afin de visualiser les noyaux des cellules. Des puits sans solution d'anticorps ont servi de contrôle négatif. Les lames ont été lues au microscope à épifluorescence (Leica®, Solms, Germany) équipé d'une caméra (DMX 1200, Nikon Europe, Badhoevedorp, Pays-Bas). Le traitement des images s'est fait à l'aide du logiciel Lucia. Les anticorps utilisés étaient les suivants : anticorps monoclonal anti-HLA-G1/-G5 (clone 87G, Exbio), anticorps polyclonaux anti-ostéocalcine (Santa Cruz ; Tebu, Le Peray en Yvelines, France), anti-ostéopontine (RnD) et anti-collagène 1a1 (Santa Cruz).

*Analyses immuno-histochimiques :*

Les échantillons biopsiques ont été fixés au formaldéhyde 10% (Sigma). Ils ont ensuite été déshydratés en utilisant des bains successifs d'éthanol à 75%, 90% et 100% (Merck et Carbo Erba, Saint Herblain, France). Ils ont enfin été placés 30 minutes dans un bain de xylène (InVitrogen) à 4°C, avant d'être inclus en paraffine (InVitrogen) pour réaliser des coupes au microtome. Les coupes ont été ensuite colorées avec de l'hématoxyline et de l'éosine. Les immuno-marquages pour la détection de l'antigène ont été réalisés en utilisant un anticorps monoclonal anti-HLA-G5/-G6 (clone 5A6G7) et un anticorps polyclonal anti-souris conjugué à la peroxydase.

*Analyses par RT-PCR :*

Après lyse cellulaire par le Trizol® (InVitrogen), 200 μL de chloroforme (Sigma) a été ajouté, afin de séparer les débris cellulaires et protéiques ainsi que l'acide désoxyribonucléique (ADN) et l'acide ribonucléique (ARN) contenu dans le surnageant. L'ARN alors récupéré a été précipité avec 500 μL d'isopropanolol (Sigma), puis lavé avec 1 mL d'éthanol à 75°C (Merck et Carbo Erba), et séché à l'air libre pendant 30 minutes, avant d'être de nouveau dissout dans 50 mL d'eau DEPC sans DNAse ou RNAse.
Le kit PrimeScript™ 1st strand cDNA Synthesis Kit Takara (Takara Bio Inc.) a été utilisé pour réaliser la synthèse d'ADN complémentaire (ADNc) par transcription inverse à partir des ARN.
Une solution d'1 μg d'ARN a été ajoutée à un premier mélange réactionnel composé d'1 μL d'amorce Oligo dT Primer, d'1 μL de dNTP Mixture, et d'eau « RNAse Free » jusqu'à un volume total du mélange de 10 μL. Après une phase de dénaturation de 5 mn à 65°C, un nouveau mélange réactionnel a été ajouté. Il était composé de 4 μL de Tampon, d'1 μL d'enzyme Prime Script™ Reverse Transcriptase, de 0,5 μL d'enzyme RNase Inhibitor et de 4,5 μL d'eau DEPC.
Le mélange final de 20 μL a alors été placé dans un thermocycleur (Applied Biosystems, Foster City, CA, USA). Les conditions utilisées étaient : hybridation amorce / ARN pendant 10 min à 30°C ; synthèse d'ADNc pendant 60 min à 42°C ; et inactivation de l'enzyme pendant 5 min à 95°C.
Le kit TaKaRa Ex Taq™ (Takara Bio Inc.) a été utilisé pour réaliser l'amplification d'une séquence d'ADN souhaitée.
Une solution d'ADNc à 50 ng/μL a été ajoutée à un mélange réactionnel composé de 2,5 μL de Tampon, de 2 μL de dNTP Mixture, de 2 μL d'amorce de l'ADN étudié, de 0,125 μL d'enzyme Taq polymerase, et d'eau DEPC jusqu'à un volume total du mélange de 25 μL. Les RT-PCR ont été réalisées selon le programme suivant : 5 min à 94°C ; 35 cycles (45sec à 94°C, 45sec à 55°C, 1 min à 72°C) ; 7 min à 70°C.
Les amorces permettant d'amplifier les différents gènes caractéristiques de la différenciation ostéogénique, de l'immaturité et de la tumorogénicité cellulaire sont présentées dans le Tableau I ci-après.

Tableau I :

| Gènes | Amorces : sens antisens |
|---|---|
| *GAPDH* | AATCCCATCACCATCTTCCAGG (SEQ ID NO : 1)<br>AGAGGCAGGGATGATGTTCTGG (SEQ ID NO : 2) |
| *BSP* | TTTCCAGTTCAGGGCAGTAGTGAC (SEQ ID NO : 3)<br>CTTCCCCTTCTTCTCCATTGTCTC (SEQ ID NO : 4) |
| *PA* | CTGGACCTCGTTGACACCTG (SEQ ID NO : 5)<br>GACATTCTCTCGTTCACCGC (SEQ ID NO : 6) |
| *α-SM actin* | TCATGATGCTGTTGTAGGTGGT (SEQ ID NO: 7)<br>CTGTTCCAGCCATCCTTCAT (SEQ ID NO : 8) |
| *PTHR1* | ACATCTGCGTCCACATCAGGG (SEQ ID NO : 9)<br>CCGTTCACGAGTCTCATTGGTG (SEQ ID NO : 10) |
| *SPARC* | ATCCCTGCCAGAACCACCACT (SEQ ID NO : 11)<br>GCGCTTCTCATTCTCATGGATCT (SEQ ID NO : 12) |
| *Osterix* | ATGGCGTCCTCCCTGCTTGAG (SEQ ID NO : 13)<br>AGGGGTGTGTCATGTCCAGAGAGG (SEQ ID NO : 14) |
| *HLA-G* | CCTTTTCAATCTGAGCTCTTCTTT (SEQ ID NO: 15)<br>GGAAGAGGAGACACGGAACA (SEQ ID NO : 16) |

## 2) Résultats

### a) Expression *in vivo* d'HLA-G par les ostéoblastes lors de la formation osseuse

Chez le nouveau né, les ostéoblastes bordant les trabécules osseux de l'os spongieux (ou moelle osseuse) expriment HLA-G5 et -G6, comme en témoigne la coloration des ostéoblastes obtenue avec l'anticorps anti-HLA-G5/-G6 (clone 5A6G7) (voir Figure 1A). A l'inverse, les chondrocytes prolifératifs et hypertrophiques n'expriment pas HLA-G5/-G6. Par ailleurs, il est à noter que certaines cellules périvasculaires de vaisseaux proches des ostéoblastes HLA-G+ (marqués par l'anticorps anti-HLA-G5/-G6) expriment également cette protéine.

Chez l'adulte sain, HLA-G5 et -G6 ont été détectés au cours de l'ostéogenèse active comme lors de la reconstruction osseuse post-fracture. Lors de la reconstruction osseuse post-fracture, au cours de la phase précoce de consolidation osseuse, les cellules mésenchymateuses se condensent pour générer des ostéoblastes capables de former de l'os. Dans cette phase précoce post-fracture, les ostéoblastes expriment faiblement HLA-G5 et -G6, alors que dans les phases plus tardives de la reconstruction osseuse, les ostéoblastes responsables de la néo-synthèse osseuse au sein du cal osseux sont très marqués par l'anticorps anti-HLA-G5/-G6 (voir Figure 1B). Par ailleurs, HLA-G5 et -G6 n'ont pas été détectées dans la moelle osseuse normale non pathologique d'un sujet (voir Figure 1C).

Ces résultats montrent que les ostéoblastes sont capables d'exprimer HLA-G. Cependant, aucune cellule HLA-G+ n'a pu être observée dans la moelle osseuse adulte non pathologique. Il ressort donc de ces résultats, qu'*in vivo,* chez l'Homme, HLA-G est exprimée par les ostéoblastes lors de la formation osseuse (ostéogenèse). Par ailleurs, des cellules périvasculaires étroitement liées aux ostéoblastes peuvent elles aussi exprimer HLA-G.

### b) HLA-G est exprimée par les ostéoblastes normaux et pathologiques

Le profil d'expression d'HLA-G5 et -G6 à partir de coupes de moelle osseuse pathologique humaine, provenant d'ostéosarcomes, d'ostéoblastomes, de sarcomes d'Ewing ou de tumeurs à cellules géantes (TCG), a été étudié.

Les résultats représentés à la Figure 2 montrent que, quelle que soit la moelle osseuse pathologique étudiée, les ostéoblastes sont marqués par l'anticorps anti-HLA-G5/-G6 (HLA-G+). Cependant, l'expression d'HLA-G5 et -G6 est plus ou moins importante en fonction de la pathologie.

Plus précisément, les ostéoblastes anormaux provenant d'ostéosarcomes et d'ostéoblastomes ainsi que les ostéoblastes normaux mais périphériques de la tumeur sont HLA-G+ (voir Figure 2A et 2B).

Sur les coupes de moelle osseuse provenant de tumeurs à cellules géantes (TCG) ou de sarcomes d'Ewing, HLA-G5 et -G6 n'ont été détectés que dans les ostéoblastes de l'environnement tumoral (voir Figure 2C).

L'expression d'HLA-G a été confirmée par l'étude de la lignée tumorale SaOs2 dérivée d'un ostéosarcome ostéoblastique. Les résultats, représentés à la Figure 3, montrent que les cellules sont marquées par l'anticorps 87G qui reconnait HLA-G5 (forme soluble d'HLA-G) et HLA-G1 (forme membranaire d'HLA-G).

Il ressort de ces résultats qu'HLA-G est exprimée par des ostéoblastes normaux et pathologiques notamment tumoraux.

c) HLA-G est exprimée par les ostéoblastes normaux générés en culture à partir de cellules souches mésenchymateuses

L'expression d'*HLA-G* par les ostéoblastes générés en culture à partir de cellules souches mésenchymateuses a été étudiée. Des cultures de cellules souches mésenchymateuses (CSMs) ont été incubées avec différents inducteurs ostéoblastiques, tels que la dexaméthasone et les « *Bone Morphogenetic Proteins* » (BMP).

L'expression des ARNm de la phosphatase alcaline (PA), de la sialo-protéine osseuse (*BSP*), du récepteur 1 de l'hormone parathyroïdienne (*PTHR1*), de l'ostéonectine (*SPARC*), du facteur de transcription osterix (*osterix*) et de l'$\alpha$-actine spécifique de la cellule musculaire lisse (*ASMA*) a été détectée dans les cellules cultivées d'origine mésenchymateuse (voir Figure 4A). L'association de ces différents transcrits est connue comme étant caractéristique des ostéoblastes (Cohen, 2006).

Les ostéoblastes ainsi obtenus exprimaient aussi les ARNm d'*HLA-G1, HLA-G2, HLA-G3, HLA-G4* et *HLA-G5* (voir Figure 4B).

Les résultats obtenus en utilisant la technique d'immunofluorescence *in situ* avec l'anticorps anti-HLA-G1/-G5 (clone 87G) sont représentés à la Figure 5. Les cellules exprimant les marqueurs ostéoblastiques étaient également positives pour HLA-G1/-G5. Cependant, il est à noter que l'expression d'*HLA-G* est significativement supérieure lorsque les ostéoblastes ont été induits par le BMP-4 comparé aux autres BMP (BMP-2 et BMP-7).

Les résultats obtenus en utilisant la technique de cytométrie en flux sont représentés à la Figure 6. La majorité des ostéoblastes expriment d'HLA-G1/-G5, tel que montré par la co-expression d'*HLA-G* et de la phosphatase alcaline (PA). Néanmoins cette expression diminue avec le temps : 66% d'ostéoblastes HLA-G+ ont été détectés après 2 semaines d'induction, alors que seulement 10% d'ostéoblastes HLA-G+ ont été détectés après 4 semaines d'induction.

Dans la mesure où les CSMs peuvent générer aussi bien des ostéoblastes que des chondroblastes et des adipocytes, il a été recherché si HLA-G pouvait être exprimée par ces autres types de cellules. Les formes solubles HLA-G5 et -G6 n'ont été détectées par ELISA que dans les surnageants des cultures ostéogéniques (cellules induites par BMP-4 et la dexaméthasone). Peu ou pas d'HLA-G soluble a été détecté dans les cultures chondrogéniques, adipogéniques et angiogéniques (Figure 7).

Par ailleurs, les chondrocytes générés en culture par la technique de micro-masse n'exprimaient pas HLA-G (Figure 8). En outre, HLA-G n'a pas été détectée dans les adipocytes.

**EXEMPLE 2: MISE EN EVIDENCE DE L'EXPRESSION D'HLA-G PAR LES OSTEOBLASTES DERIVES D'OSTEOSARCOMES**

**1) Matériel et Méthode**

*Cellules :*

Lignées d'ostéosarcomes : HOS-154732 (McAllister *et al.,* 1971), U2OS (Ponten *et al.,* 1967), MG-63 (Billiau *et al.,* 1977), SaOS2 (Fogh *et al.,* 1975), CAL72 (Rochet *et al.,* 1999) et SaOS2 RUNX2 DNN (Ghali *et al.,* 2010).

Les différentes biopsies étudiées provenaient du service d'Anatomie Pathologique du Centre Hospitalier Universitaire (CHU) Trousseau de Tours (France).

*Cellules souches mésenchymateuses (CSM) :*

Les CSM utilisées provenaient de donneurs sains, pris en charge dans le service de Chirurgie Orthopédique et Traumatologique du CHU de Tours (France) et hospitalisés pour la mise en place d'une prothèse totale de hanche. Un consentement éclairé du patient a été recueilli par écrit. 20 mL de moelle osseuse ont été prélevés dans la crête iliaque postérieure lors de l'intervention. Ces cellules ont servi de contrôle pour tous les tests effectués dans cette étude.

*Cultures cellulaires :*

- Culture standard : les cellules des différentes lignées ont été cultivées en flacons de culture (Falcon®, BD Biosciences, VWR, Strasbourg, France), à la densité initiale de 5000 cellules par cm$^2$. Le milieu de culture est composé de Minimum Essential Médium alphaMEM (InVitrogen), avec 10% (v/v) de sérum de veau foetal (SVF) (Perbio Hyclone, Logan, USA), 1% (v/v) de L-glutamine, 1% (v/v) de pénicilline et streptomycine (InVitrogen) et 100 µM de fungizone (Bristol Myers Squibb, Rueil Malmaison, France). Le milieu de culture a été changé tous les 2 à 3 jours.
- Les CSMs ont été cultivées de manière identique avec le même milieu de culture, contenant en outre du FGF2 (AbCys, Paris, France) à 1 ng/mL.
- Milieu de différenciation : pour la différenciation ostéocytaire, le milieu est composé d'alphaMEM complémenté par 2 % (v/v) de SVF et 50 ng/mL de BMP4 (Peprotech, Londre UK). La durée de cette culture est de 8 à 10 jours.

*Cytométrie en flux :*

200 000 cellules ont été marquées à l'aide d'un anticorps couplé à un fluorochrome : la phycoérythrine (PE) ou la fluorescéine isothiocyanate (FITC). Pour mettre en évidence les antigènes exprimés à la surface des cellules, les cellules isolées et vivantes ont été incubées 45 minutes avec les anticorps reconnaissant ces antigènes, lavées puis fixées au Cell Fix™ (Becton Dickinson, Erembodegem, France). Pour mettre en évidence les antigènes intracellulaires, une étape préalable de perméabilisation cellulaire au CytoFix/CytoPerm™ (Becton Dickinson) est nécessaire avant l'immunomarquage. Le témoin négatif a été obtenu à l'aide d'une immunoglobuline aspécifique.

Les cellules ont ensuite été passées au cytomètre (FACS Calibur™, Becton Dickinson) à laser Argon émettant à la longueur d'onde de 488 nm. L'interprétation du résultat a été réalisée à l'aide du logiciel CellQuest 3.1™.

Les anticorps anti-HLA-G1 et -G5 87G conjugué à l'alexa488 et MEM/G9 conjugué à l'APC ou le 4H84 (Exbio ; Praha ; République Tchèque), et un anticorps anti-ALPL (R&D System) ont été utilisés.

*Analyses par RT-PCR :*

• Extraction au Trizol® :

Après lyse cellulaire à l'aide du Trizol® (InVitrogen) et ajout de 200 µL de chloroforme (Sigma) puis centrifugation, trois phases ont été obtenues : la phase inférieure contenant les protéines, la phase intermédiaire contenant les acides désoxyribonucléiques (ADN) et la phase supérieure contenant les acides ribonucléiques (ARN). Cette dernière a été extraite puis précipitée dans 500 µL d'isopropanol (Sigma), puis lavée avec 1 mL d'éthanol 75% (Merck et Carbo Erba) et laissée séchée à l'air ambiant jusqu'à l'obtention d'une transparence complète. Enfin, l'ARN a été dissous dans 50 mL d'eau diéthylpyrocarbonate (DEPC - pour inhiber les ARNases).

• Dosage des ARN :

Deux microlitres d'ARN ont été dilués dans 98 µL d'eau DEPC, puis placés dans une cuve en quartz du dosimètre Gene Quant II (Amersham Pharmacia, Sarclay, Orsay, France), après réalisation d'un blanc (eau seule). Le dosage se faisait par mesure de la densité optique à l'aide d'un laser émettant à 260 nm.

• Transcription inverse :

1 µg d'ARN a été dilué dans de l'eau DEPC jusqu'à l'obtention de 8 µL. Le Random Hexamer et les dNTP (kit PrimeScript™ 1st stand cDNA synthesis Kit Takara (Takara Bio Inc.)) ont été ajoutés. Après dénaturation (5 min à 65°C) au thermocycleur (Applied Biosystems, Foster City, CA, USA), l'enzyme Prime Script™ Reverse transcriptase a été ajoutée. Cette solution a été incubée dans le thermocycleur, suivant le programme suivant : première étape de 10 min à 30°C, deuxième étape de 60 min à 42°C, puis troisième étape de 5 min à 95°C. Les ADN complémentaires (ADNc) obtenus ont été stockés à -20°C.

• Réaction de Polymérisation en Chaîne (PCR) :

25 ng d'ADNc ont été mélangés aux dNTP, aux amorces ciblant une séquence d'intérêt (voir Tableau II ci-après) et à l'enzyme Taq Polymerase (kit TaKara™ Ex Taq). Puis le tout a été incubé dans le thermocycleur, suivant le programme suivant composé de 35 cycles, chaque cycle étant constitué d'1 min à 98°C, suivie de 30 secondes à 55°C, suivie de 1 min à 72°C.

Tableau II :

| Gènes | Amorces : sens antisens |
|---|---|
| *GAPDH* | ATCCCATCACCATCTTCCAGG (SEQ ID NO : 17)<br>GAGGCAGGGATGATGTTCTGG (SEQ ID NO : 18) |
| *ALPL* (ou *PA*) | CTGGACCTCGTTGACACCTG (SEQ ID NO : 5)<br>GACATTCTCTCGTTCACCGC (SEQ ID NO : 6) |
| *Runx2* | GGCCCACAAATCTCAGATCGTT (SEQ ID NO : 19)<br>CACTGGCGCTGCAACAAGAC (SEQ ID NO : 20) |
| *Dlx5* | GCCACCAACCAGCCAGAGAA (SEQ ID NO : 21)<br>GCGAGGTACTGAGTCTTCTGAAACC (SEQ ID NO : 22) |
| *Ostéonectine* (*SPARC*) | ATCCCTGCCAGAACCACCACT (SEQ ID NO : 11)<br>GCGCTTCTCATTCTCATGGATCT (SEQ ID NO : 12) |
| *Collα1* | ACATGGACCAGCAGACTGGCA (SEQ ID NO : 23)<br>TCACTGTCTTGCCCCAGGCT (SEQ ID NO : 24) |

• Migration sur gel d'agarose :

Les produits de PCR ont été déposés sur un gel à 1 % d'agarose (Sigma) contenant 0,01 % de Bromure d'Ethidium (Sigma). Le gel a ensuite été révélé à l'aide d'une lampe à ultra violet (Vilbert Lourmat, Eberhardzell, Deutschland). Puis les bandes ont été analysées à l'aide du logiciel Chemicapt™ (BioRad, CA, USA).

• Réaction de Polymérisation en Chaîne quantitative (qPCR) :

50 ng d'ADNc ont été mélangés à une solution contenant une molécule fluorescente (Syber Green, In Vitrogen). Cette solution a ensuite été placée dans un puits (plaque 96 puits) dans lequel les amorces ont été préalablement déposées.

La plaque a été placée dans le thermocycleur (BioRad). Le programme suivant a été utilisé : 30 secondes à 95°C, 30 secondes à 56°C, 72°C pendant 30 secondes, 39 cycles durant. Une courbe de fusion a été réalisée afin de vérifier la qualité des ADN amplifiés.

La quantité d'ADN a été évaluée grâce à la formule suivante :

$$2^{-(C(t)\ du\ gène\ -\ C(t)\ GAPDH)} \quad ou \quad 2^{-\Delta c(t)}$$

• Western blot :

Les cellules ont été décollées, centrifugées puis reprises dans 500 μL de tampon de lyse composé de Triton X 100 à 0,1 % (v/v) (Sigma). Après centrifugation, le surnageant a été prélevé et les extraits protéiques ont ensuite été dilués dans du tampon Laemlli puis portés à ébullition pendant 2 minutes.

Les échantillons ont été dosés par mesure de la densité optique à l'aide du MRX II (Dynex technologies, Chantilly, USA), selon la technique de Bradford.

L'électrophorèse a été réalisée sur gel de polyacrylamide avec différentes concentrations en fonction de la protéine d'intérêt (10 % pour la Phosphatase Alcaline et HLA-G), dans un tampon de Sodium Dodécyl Sulfate (SDS - Biorad). Les protéines ont été transférées sur une membrane de polyfluorure de vinylidène, qui a ensuite été saturée par des protéines de lait et incubée avec l'anticorps d'intérêt pendant une nuit. Après application de l'anticorps secondaire, la révélation a été effectuée par chémoluminescence (kit ECL, Amersham).

• Transfection :

Les cellules ont été transfectées par 3 siRNA différents (InVitrogen) ciblant *RUNX2* (Select RNAi™ siRNA, Catalogue No 1299003) ou *DLX5* (Stealth Select RNAi™ siRNA, Catalogue No 1299003). Des siRNA non spécifiques ont été utilisés comme contrôles (InVitrogen). 20 nM de siRNA ont été utilisés pour les transfections qui ont été réalisées à l'aide du kit Amaxa Nucleofactor Kit (Lonza, France), conforméménent aux instructions du fournisseur. Après 24 heures, les cellules ont été induites à se différencier dans le milieu de différentiation ostéogénique. Après 2, 4 et 6 jours les cellules ont été récoltées et testées pour leur expression de gènes caractéristiques des ostéoblastes ou codant HLA-G.

## 2) Résultats

L'expression par cytométrie en flux et par immuno-marquage (*Western blotting*) des isoformes HLA-G1 (HLA-G membranaire) HLA-G5 (HLA-G intracellulaire) dans différentes lignées ostéoblastiques d'origine humaine CAL72, MG-63, HOS et U2OS a été étudiée. Les résultats sont représentés à la Figure 9. Les isoformes HLA-G1 et HLA-G5 ont été détectées dans toutes les lignées mais à des degrés d'expression divers. Il a été observé qu'HLA-G5 était toujours fortement exprimé alors que HLA-G1 l'était seulement pour les lignées CAL72 et MG-63 ; HOS et U2OS l'exprimant significativement moins.

Les résultats de la recherche de l'expression d'HLA-G (HLA-G1 et HLA-G5) par immuno-histochimie dans les tissus osseux normaux (au niveau de la zone de croissance des os) et pathologiques d'origine humaine (chez l'adulte) sont représentés dans le Tableau III ci-dessous. Il ressort de ce tableau que seuls les ostéoblastes normaux ou tumoraux et certains chondrocytes hypertrophiques expriment HLA-G1 et -G5.

Tableau III : Expression d'HLA-G1 et -G5 dans les tissus osseux normaux ou pathologiques humains.

| Tissus | Expression d'HLA-G |
|---|---|
| **Tissus normaux** | |
| endothélium de la moelle osseuse | - |
| ostéoclastes | - |
| ostéoblastes trabéculaires | + |
| ostéoblastes corticaux | + |
| chondrocytes de la zone de repos | - |
| chondrocytes de la zone proliférative | - |
| chondrocytes de la zone hypertrophique | ±[a] |
| adipocytes de la moelle osseuse | - |
| Cellules vasculaires musculaires lisses et péricytes | +[b] |
| cellules mésenchymateuses du périoste | + |
| cellules mésenchymateuses du périchondre | + |
| **Tissus tumoraux** | |
| ostéosarcomes ostéoblastiques | + |
| ostéosarcomes fibroblastiques | ± |
| **ostéoblastomes** | + |
| chondrosarcomes | ± |
| chondroblastomes | - |
| cellules de tumeur Ewing | - |
| tumeurs à cellules géantes (TCG) | - |
| ostéoblastes normaux induits par la tumeur | +[c] |
| [a] : certains chondrocytes expriment HLA-G<br>[b] : les cellules périvasculaires HLA-G positives dans les sites actifs de formation osseuse<br>[c] : les ostéoblastes normaux induits par les tumeurs osseuses sont tous HLA-G positifs | |

Pour confirmer la spécificité d'expression d'HLA-G par les ostéoblastes, l'expression des gènes *DLX5* et *RUNX2* impliqués dans l'induction de l'ostéogénèse a été inhibée par ARN interférence. Après transfection des cellules souches mésenchymateuses de moelle osseuse d'origine humaine par des siRNA ciblant *DLX5* ou *RUNX2,* les cellules ont été cultivées dans un milieu ostéogénique (ajout de BMP4). Pendant 6 jours, l'expression de gènes caractéristiques des ostéoblastes (phosphatase alcaline ou *ALPL,* l'ostéonectine ou *SAPRC,* le collagène 1$\alpha$1 ou *COL1$\alpha$1*) ainsi qu'*HLA-G* a été étudiée. Il a été observé (voir Figure 10) que la diminution de l'expression de *DLX5* et *RUNX2* engendraient une diminution de l'expression des gènes *ALPL, SPARC, COL1$\alpha$1* mais aussi *d'HLA-G.*

Pour confirmer ces résultats, 2 lignées SaOS2 qui expriment une forme inactivante de RUNX2 (*RUNX2* dominant négatif ou DNN) ont été utilisées : les lignées $\Delta$6A2 et $\Delta$4A5. La lignée transduite ne contenant pas *RUNX2 DNN* a servie de lignée contrôle (C-). $\Delta$4A5 exprime fortement le DNN RUNX2 alors que son expression est plus modérée chez $\Delta$6A2. Les résultats sont représentés à la Figure 11.

Dans un premier temps, l'expression du COL1$\alpha$1 a été étudiée par PCR quantitative (QRT-PCR) et *Western blot* dans la mesure où il est induit par RUNX2. Il a alors été détecté une diminution de l'expression de COL1$\alpha$1 au niveau de l'ARN et au niveau protéique dans les lignées RUNX2 DNN. Ces diminutions étaient significativement plus importantes dans la lignée $\Delta$4A5 que dans la $\Delta$6A2.

L'expression d'HLA-G (isoformes HLA-G1 et HLA-G5) a ensuite été étudiée par cytométrie en flux et *Western blot.* L'expression d'HLA-G a été trouvée quasiment éteinte dans $\Delta$4A5 et plus faiblement exprimée dans $\Delta$6A2 que dans la lignée contrôle C-.

## Bibliographie

- Betts MR et al., J Immunol. Methods, 281:65-78 (2003).
- Billiau A et al., Antimicrob Agents Chemother., 12:11-15 (1977).
- Carosella ED et al., Blood, 111:4862-4870 (2008a).
- Carosella ED et al., Trends Immunol., 29:125-132 (2008b)
- Cohen MM, Am J Med Genetics Part A, 140A:2646-2706 (2006).
- Ellis SA et al., J. Immunol., 144:731-735 (1990).
- Fogh J *et al., New York: Plénum Press,* 115-159 (1975).
- Friedenstein AJ et al., Exp Hematol., 4:267-274 (1976).
- Geraghty DE et al., Proc Natl Acad Sci. USA, 84:9145-9149 (1987).
- Ghali O et al., Bone, 46:901-910 (2010).
- Hackmon R et al., Fetal Diagn Ther., 19:404-409 (2004).
- Ishitani A and Geraghty DE., Proc Natl Acad. Sci. USA, 89:3947-3951 (1992).
- Kirszenbaum M et al., Proc Natl Acad Sci. USA, 91:4209-4213 (1994).
- Kirszenbaum M et al., Human Immunol., 43:237-241 (1995).
- Le Discorde M et al. Biol Reprod., 73:280-288 (2005).
- Le Rond S et al., Eur J Immunol., 34:649-660 (2004).
- Le Rond S et al., J. Immunol., 176:3266-3276 (2006).
- McAllister RM et al., Cancer, 27:397-402 (1971).
- McMaster M et al., J Immunol., 160:5922-5928 (1998).
- Menier C et al., Hum Immunol., 64:315-26 (2003).
- Moreau P et al., Human Immunol., 43:231-236 (1995).
- Naji A, et al., Blood, 110:3936-3948 (2007).
- Pittenger MF et al., Science, 284:143-147 (1999).
- Ponten J et al., Int J Cancer, 2:434-447 (1967).
- Rebmann V et al., Tissue Antigens, 53:14-22 (1999).
- Rebmann V et al., Human Immunology, 66: 853-863 (2005).
- Rochet N et al., Int J Cancer, 82:282-285 (1999).
- Rouas-Freiss N et al., Cancer Res., 65:10139-10144 (2005).
- Selmani Z et al., Stem Cells, 26:212-222 (2008).
- Srivastava A, Medscape from Curr Med Res Opin., 21:1015-1026 (2005).
- Ugurel S et al., Cancer, 92:369-376 (2001).
- Vesper H, Medscape from Lab Med., 36:424-429 (2005).

**Revendications**

1.  Méthode *in vitro* de suivi de l'évolution d'une tumeur osseuse chez un sujet, à partir d'échantillons biologiques comprenant des ostéoblastes, obtenus par biopsie osseuse de ladite tumeur à un temps t0 et à un temps t1, **caractérisée en ce qu'**elle comprend une étape de détermination de la concentration ou de détermination quantitative de l'expression des isoformes HLA-G1 et HLA-G5 dans lesdits échantillons biologiques,

    - une augmentation de la concentration ou du niveau d'expression d'au moins une desdites isoformes d'HLA-G entre les temps t0 et t1 étant l'indication d'une progression de ladite tumeur osseuse chez ledit sujet,
    - une diminution de la concentration ou du niveau d'expression d'au moins une desdites isoformes d'HLA-G entre les temps t0 et t1 étant l'indication d'une rémission de ladite tumeur osseuse chez ledit sujet.

2.  Méthode selon la revendication 1, **caractérisée en ce que** ledit sujet est un humain.

3. Méthode selon la revendication 1 ou la revendication 2, **caractérisée en ce que** l'on détermine quantitativement l'expression par les ostéoblastes d'au moins une desdites isoformes d'HLA-G, à partir d'échantillons biologiques comprenant des ostéoblastes.

4. Méthode selon l'une quelconque des revendications 1 à 3, **caractérisée en ce que** l'on détermine ladite concentration ou ladite expression d'au moins une desdites isoformes d'HLA-G dans lesdits échantillons biologiques par une méthode immunologique appropriée.

5. Méthode selon la revendication 4, **caractérisée en ce que** l'on détermine quantitativement l'expression par les ostéoblastes d'au moins une desdites isoformes d'HLA-G par la détection des ARNm codant ladite isoforme d'HLA-G.

6. Méthode selon l'une quelconque des revendications 1 à 5, **caractérisée en ce que** ladite tumeur osseuse est choisie parmi l'ostéosarcome, l'ostéoblastome, le sarcome d'Ewing et les tumeurs à cellules géantes.

**Patentansprüche**

1. In-vitro-Methode zur Verfolgung der Entwicklung eines Knochentumors bei einem Probanden auf der Basis von biologischen Proben, die Osteoblasten umfassen, erhalten durch Knochenbiopsie des Tumors zu einer Zeit t0 und zu einer Zeit t1, **dadurch gekennzeichnet, dass** sie einen Schritt der Bestimmung der Konzentration oder der quantitativen Bestimmung der Expression der Isoformen HLA-G1 und HLA-G5 in den biologischen Proben umfasst,

- wobei eine Erhöhung der Konzentration oder des Expressionsniveaus von mindestens einer der HLA-G-Isoformen zwischen den Zeiten t0 und t1 der Hinweis eines Fortschreitens des Knochentumors bei dem Probanden ist,
- wobei eine Verringerung der Konzentration oder des Expressionsniveaus von mindestens einer der HLA-G-Isoformen zwischen den Zeiten t0 und t1 der Hinweis auf eine Remission des Knochentumors bei dem Probanden ist.

2. Methode nach Anspruch 1, **dadurch gekennzeichnet, dass** der Proband ein Mensch ist.

3. Methode nach Anspruch 1 oder Anspruch 2, **dadurch gekennzeichnet, dass** die Expression durch die Osteoblasten von mindestens einer der HLA-G-Isoformen auf der Basis von biologischen Proben, die Osteoblasten enthalten, quantitativ bestimmt wird.

4. Methode nach einem der Ansprüche 1 bis 3, **dadurch gekennzeichnet, dass** die Konzentration oder die Expression von mindestens einer der HLA-G-Isoformen in den biologischen Proben durch eine geeignete immunologische Methode bestimmt wird.

5. Methode nach Anspruch 4, **dadurch gekennzeichnet, dass** die Expression durch die Osteoblasten von mindestens einer der HLA-G-Isoformen durch die Detektion der mRNA, welche die HLA-G-Isoform codiert, quantitativ bestimmt wird.

6. Methode nach einem der Ansprüche 1 bis 5, **dadurch gekennzeichnet, dass** der Knochentumor aus dem Osteosarkom, dem Osteoblastom, dem Ewing-Sarkom und den Riesenzellentumoren ausgewählt ist.

**Claims**

1. *In vitro* method for monitoring the development of a bone tumour in a subject, from biological samples comprising osteoblasts obtained via bone biopsy of said tumour at a time t0 and a time t1, **characterized in that** it comprises a step of determining the concentration or of determining quantitatively the expression of HLA-G1 and HLA-G5 isoforms in said biological samples:

- an increase in the concentration or in the level of expression of at least one of said HLA-G isoforms between times t0 and t1 being an indication of progression of said bone tumour in said subject;
- a decrease in the concentration or in the level of expression of at least one of said HLA-G isoforms between times t0 and t1 being an indication of remission of said bone tumour in said subject.

2. The method according to claim 1, **characterized in that** said subject is a human subject.

3. The method according to claim 1 or claim 2, **characterized in that** expression by the osteoblasts of at least one of said HLA-G isoforms is determined quantitatively from biological samples comprising osteoblasts.

4. The method according to any of claims 1 to 3, **characterized in that** said concentration or said expression of at least one of said HLA-G isoforms in said biological samples are determined by a suitable immunological method.

5. The method according to claim 4, **characterized in that** the expression by the osteoblasts of at least one of said HLA-G isoforms is determined quantitatively by detection of the mRNAs encoding said HLA-G isoform.

6. The method according to any of claims 1 to 5, **characterized in that** said bone tumour is selected from among osteosarcoma, osteoblastoma, Ewing's sarcoma and giant cell tumours.

A  Zone de croissance osseuse

Neg control

B

C  Cal précoce     Neg control     Cal tardif     Neg control

Moelle osseuse adulte

**Figure 1**

A

Ostéosarcomes

B

Ostéoblastomes

Neg controls

C

Ewing     TCG

**Figure 2**

Figure 3

Figure 4

Figure 5

**Figure 6**

**Figure 7**

**Figure 8**

Figure 9

**Figure 10**

A

COL1a1

B

C

**Figure 11**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007104724 A
- EP 0677582 A

- FR 0905624

**Littérature non-brevet citée dans la description**

- **BETTS MR et al.** *J Immunol. Methods,* 2003, vol. 281, 65-78
- **BILLIAU A et al.** *Antimicrob Agents Chemother.,* 1977, vol. 12, 11-15
- **CAROSELLA ED et al.** *Blood,* 2008, vol. 111, 4862-4870
- **CAROSELLA ED et al.** *Trends Immunol.,* 2008, vol. 29, 125-132
- **COHEN MM.** *Am J Med Genetics Part A,* 2006, vol. 140A, 2646-2706
- **ELLIS SA et al.** *J. Immunol.,* 1990, vol. 144, 731-735
- **FRIEDENSTEIN AJ et al.** *Exp Hematol.,* 1976, vol. 4, 267-274
- **GERAGHTY DE et al.** *Proc Natl Acad Sci. USA,* 1987, vol. 84, 9145-9149
- **GHALI O et al.** *Bone,* 2010, vol. 46, 901-910
- **HACKMON R et al.** *Fetal Diagn Ther.,* 2004, vol. 19, 404-409
- **ISHITANI A ; GERAGHTY DE.** *Proc Natl Acad. Sci. USA,* 1992, vol. 89, 3947-3951
- **KIRSZENBAUM M et al.** *Proc Natl Acad Sci. USA,* 1994, vol. 91, 4209-4213
- **KIRSZENBAUM M et al.** *Human Immunol.,* 1995, vol. 43, 237-241
- **LE DISCORDE M et al.** *Biol Reprod.,* 2005, vol. 73, 280-288
- **LE ROND S et al.** *Eur J Immunol.,* 2004, vol. 34, 649-660
- **LE ROND S et al.** *J. Immunol.,* 2006, vol. 176, 3266-3276

- **MCALLISTER RM et al.** *Cancer,* 1971, vol. 27, 397-402
- **MCMASTER M et al.** *J Immunol.,* 1998, vol. 160, 5922-5928
- **MENIER C et al.** *Hum Immunol.,* 2003, vol. 64, 315-26
- **MOREAU P et al.** *Human Immunol.,* 1995, vol. 43, 231-236
- **NAJI A et al.** *Blood,* 2007, vol. 110, 3936-3948
- **PITTENGER MF et al.** *Science,* 1999, vol. 284, 143-147
- **PONTEN J et al.** *Int J Cancer,* 1967, vol. 2, 434-447
- **REBMANN V et al.** *Tissue Antigens,* 1999, vol. 53, 14-22
- **REBMANN V et al.** *Human Immunology,* 2005, vol. 66, 853-863
- **ROCHET N et al.** *Int J Cancer,* 1999, vol. 82, 282-285
- **ROUAS-FREISS N et al.** *Cancer Res.,* 2005, vol. 65, 10139-10144
- **SELMANI Z et al.** *Stem Cells,* 2008, vol. 26, 212-222
- **SRIVASTAVA A.** *Medscape from Curr Med Res Opin.,* 2005, vol. 21, 1015-1026
- **UGUREL S et al.** *Cancer,* 2001, vol. 92, 369-376
- **VESPER H.** *Medscape from Lab Med.,* 2005, vol. 36, 424-429